**Europäisches Patentamt**

**European Patent Office**

**Office européen des brevets**

(19)

(11) Veröffentlichungsnummer : **0 361 068 B1**

(12) **EUROPÄISCHE PATENTSCHRIFT**

(45) Veröffentlichungstag der Patentschrift :
**09.12.92 Patentblatt 92/50**

(51) Int. Cl.⁵ : **G01N 1/34,** G01N 33/00,
B01D 53/00, B01D 5/00

(21) Anmeldenummer : **89115322.3**

(22) Anmeldetag : **19.08.89**

(54) **Vorrichtung zur Abscheidung dampfförmiger Bestandteile aus einem Gasstrom.**

(30) Priorität : **30.09.88 DE 3833192**

(43) Veröffentlichungstag der Anmeldung :
**04.04.90 Patentblatt 90/14**

(45) Bekanntmachung des Hinweises auf die
Patenterteilung :
**09.12.92 Patentblatt 92/50**

(84) Benannte Vertragsstaaten :
**AT BE CH ES FR GB GR IT LI LU NL SE**

(56) Entgegenhaltungen :
EP-A- 0 291 630
CH-A- 360 373
DE-C- 96 296
US-A- 3 368 386

(73) Patentinhaber : **Gröger & Obst Mess- und
Regeltechnik GmbH
Marienstrasse 4
W-8137 Berg 2 (DE)**

(72) Erfinder : **Gröger, Christa-Maria
Primelweg 1
W-8137 Berg 3 (DE)**
Erfinder : **Gröger, Helmut
Primelweg 1
W-8137 Berg 3 (DE)**

(74) Vertreter : **LOUIS, PÖHLAU, LOHRENTZ &
SEGETH
Ferdinand-Maria-Strasse 12
W-8130 Starnberg (DE)**

EP 0 361 068 B1

**Beschreibung**

Die Erfindung betrifft eine Vorrichtung zur Abscheidung dampfförmiger Bestandteile aus einem Gasstrom durch Kondensation bei der Gasaufbereitung, insbesondere für die Gasanalyse, mit den Merkmalen gemäß dem Oberbegriff des Patentanspruches 1.

Im Rahmen der Gasaufbereitung zum Zweck der Gasanalyse ist es erforderlich, aus dem zu analysierenden Gas zunächst solche Bestandteile abzuscheiden, die die Zielsubstanz bzw. Zielsubstanzen in dem Gasstrom begleiten, für die Messung jedoch hinderlich sind und/oder das Meßergebnis sogar verfälschen. Zu derartigen Bestandteilen zählt regelmässig Wasser in Dampfform, das deshalb aus dem Gasstrom abgeschieden werden muß. Für den überwiegenden Teil des in dem Gasstrom enthaltenen Wasserdampfes erfolgt das normalerweise durch eine Abkühlung des Gasstromes auf eine nur wenig über 0°C liegende Temperatur, wodurch der Wasserdampf kondensiert und das Kondensat abgeführt werden kann. Eine diesem Zweck dienende bekannte Kühlvorrichtung (DE-GBM 87 07 036) besteht im wesentlichen aus einem von einem Peltier-Kühlelement direkt von außen gekühlten, weitgehend aufrecht angeordneten Kühlrohr, in welchem konzentrisch ein Innenrohr angeordnet ist, das einen oben liegenden Gaseinlaß bildet und kurz vor dem kegelig zulaufenden Unterteil des Kühlrohres frei mündet. Das untere Ende des Kühlrohres ist zu einem Kondensatablaufstutzen geformt; der Gasauslaß befindet sich als seitlicher Anschlußstutzen in der Nähe des oberen Endes des Kühlrohres. Durch diese Konstruktion strömt das aufzubereitende Gas zunächst durch das Innenrohr und wird dann zwischen diesem und der gekühlten Außenwand des Kühlrohres im Gegenstrom wieder nach oben geführt und durch den Gasauslaß abgezogen. In dem Gasstrom enthaltener Wasserdampf schlägt sich an der gekühlten Außenwand nieder und das Kondensat läuft nach unten in den Kondensatablaufstutzen.

Diese bekannte Kühlvorrichtung ist nicht frei von Nachteilen: Infolge des seitlichen Anschlusses des Gasauslasses durchströmt das am unteren Ende des Innenrohres austretende, noch relativ warme Gas allenfalls im unteren Endbereich des Kühlrohres dessen ganzen Querschnitt, beschränkt sich aber im weiteren Verlauf des Gegenstromes auf einen immer geringer werdenden Querschnitt auf der Seite, auf der der Gasauslaß liegt. Dadurch bildet sich ein von dem Gas nicht durchsetzter Totraum auf der dem Gasauslaß gegenüberliegenden Seite des Kühlrohres, der für die Gaskühlung nicht oder kaum genutzt wird. Da somit die tatsächlich genutzte Kühlfläche geringer als die rechnerische Kühlfläche ist, muß eine hohe Kühlleistung erbracht werden, um eine hinreichende Abscheidung des in dem Gasstrom enthaltenen Wasserdampfes zu bewirken. Eine weitere Steigerung der Kühlleistung und damit eine Verringerung des Kühl-Wirkungsgrades rührt daher, daß das Innenrohr auf seiner gesamten Länge allenfalls indirekt, nämlich durch den im Gegenstrom ansteigenden Gasstrom gekühlt ist und daher der zum Gasauslaß geführte Gasstrom einseitig, nämlich von der Außenwand des Innenrohres, beheizt wird. Daraus resultiert auch, daß sich ein stationärer Zustand, in welchem ein Temperaturgleichgewicht herrscht, erst nach relativ langer Zeit nach dem Anfahren des Gasaufbereitungsgerätes einstellt und die am Gasauslaß erzielte Gastemperatur starken Regelschwankungen unterliegt, wenn zum Zweck ihrer Einstellung die Kühlleistung verändert wird. Dies kann sogar dazu führen, daß das Kondensat an der Innenwand des Kühlrohres anfriert, so daß der Strömungsquerschnitt verstopft und die Kühlvorrichtung damit blockiert wird. Schließlich besteht ein wesentlicher Nachteil darin, daß durch den Kontakt des Gases mit dem kräftigen Kondensatfilm, der durch die geschilderte Konstruktion an der Innenwand des Kühlrohres entsteht, weitere Bestandteile und vor allem Anteile der Zielsubstanz bzw. Zielsubstanzen aus dem Gas ausgewaschen werden, wodurch das Meßergebnis verfälscht wird.

Aufgabe der Erfindung ist es daher, eine Vorrichtung der eingangs geschilderten Art zu schaffen, die einen hohen Kühl-Wirkungsgrad ergibt, ein besseres Regelverhalten zeigt und keine oder nur eine geringe Auswaschung der Zielsubstanz zur Folge hat. Außerdem soll die Vorrichtung einfach aufgebaut sein.

Erfindungsgemäß wird diese Aufgabe gelöst durch die Ausgestaltung gemäß dem Kennzeichen des Patentanspruches 1.

Bei der erfindungsgemässen Vorrichtung wird auf eine Gegenstromführung des Gasstromes gerade verzichtet und statt dessen eine Durchströmung des Kühlrohres von unten nach oben erzeugt, wobei der Gasstrom jedoch infolge der tangentialen Zuführung am unteren Ende das Kühlrohr der Länge nach schraubenförmig durchsetzt und auf diese Weise auf einem längeren Weg mit diesem in Kontakt kommt. Dieser Kontakt wird dadurch aufrecht erhalten bzw. intensiviert, daß sich der Querschnitt des Kühlrohres zum Gasauslaß hin verringert, um dadurch dem mit absinkender Gastemperatur geringeren Gasvolumen Rechnung zu tragen. Infolgedessen kommt das Gas mit der gesamten gekühlten Fläche des Kühlrohres in Kontakt, so daß im Vergleich zu der eingangs geschilderten bekannten Kühlvorrichtung die rechnerische Kühlfläche verringert werden kann und trotzdem bei gleichem Abscheideeffekt eine geringere Kühlleistung erforderlich ist. Die bessere Ausnutzung der Kühlfläche führt auch zu einem gleichmässigeren dünneren Kondensatfilm, der eine geringere Auswaschung zur Folge hat. Schließlich ist infolge des einfacheren Aufbaues die erfindungsgemässe Vorrichtung einfacher herzustellen und daher billiger.

Da die Verjüngung des Kühlrohres nach oben hin einen Ausgleich der durch die Abkühlung bewirkten Volumensverminderung des Gasstromes erreichen soll, sollte im Idealfall der Querschnittsverlauf auf den in der Regel nichtlinearen temperaturabhängigen Volumensschwund abgestimmt sein. Jedoch hat sich gezeigt, daß durch eine konische Verjüngung eine sehr weitgehende Anpassung an diesen Idealfall erzielbar ist, wobei vorzugsweise ein Konuswinkel in der Grössenordnung von 5° eingehalten wird. Die konische Ausführung erleichtert nicht nur die Erzeugung des Kühlrohres, sondern vereinfacht auch die Herstellung eines innigen wärmeübertragenden Kontaktes zwischen dem Kühlelement und der Kühlrohraußenwand. Dieses Kühlelement, das vorzugsweise ein metallischer Kontaktblock mit einem Peltier-Element ist, kann aufgrund der konischen Ausführung des Kühlrohres einfach ohne besondere Befestigungsmaßnahmen auf diesem aufsitzen, ohne daß die Gefahr eines Kontaktverlustes besteht.

Weitere Vorteile und Merkmale der Erfindung ergeben sich aus den Unteransprüchen.

Ein Ausführungsbeispiel der Erfindung ist nachfolgend anhand der beiliegenden Zeichnungen näher erläutert. In den Zeichnungen zeigen:

Fig. 1 eine Seitenansicht, teilweise geschnitten längs der Linie I-I in Fig. 2, eines erfindungsgemässen Kühlrohres, und

Fig. 2 eine Draufsicht auf das Kühlrohr von oben.

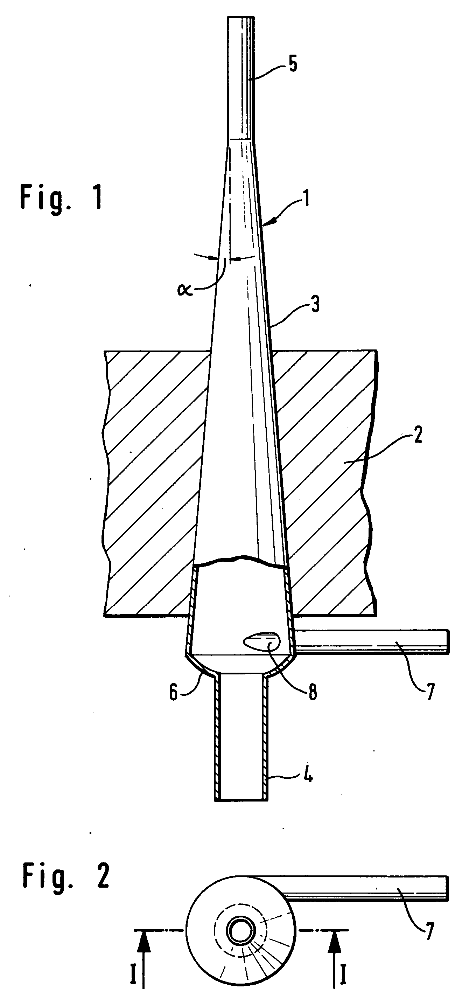

Gemäß der Darstellung in Fig. 1 besteht die erfindungsgemässe Abscheidevorrichtung im wesentlichen aus einem im Ganzen mit 1 bezeichneten Kühlrohr und einem nur angedeuteten Kühlelement 2. Das Kühlrohr 1 besitzt einen sich von unten nach oben konisch verjüngenden Kühlabschnitt 3, an den unten ein Kondensatablaufstutzen 4 und oben ein zylindrischer Gasauslaßstutzen 5 jeweils koaxial anschließen. Der Kondensatablaufstutzen 4 geht von einer muldenförmigen Bodenwand 6 des Kühlabschnittes 3 aus, und unmittelbar über der Bodenwand 6 mündet bei 8 in das Innere des Kühlabschnittes 3 ein Gaseinlaßstutzen 7, der tangential zum Querschnitt des Kühlabschnittes 3 an dieser Stelle angeordnet ist und dessen Achse senkrecht zur Achse des Kühlrohres 1 verläuft, diese jedoch kreuzt.

Die äußere Mantelfläche des Kühlabschnittes 3 ist weitgehend exakt konisch und weist einen Kegelwinkel α von etwa 5° auf. Das Kühlelement 2, das aus einem Aluminium-Kontaktblock mit einem nicht dargestellten Peltier-Element sein kann, weist eine der Konizität des Kühlabschnittes 3 möglichst genau angepasste Bohrung auf, so daß es - wie aus Fig. 1 ersichtlich ist - auf das Kühlrohr 1 aufgesetzt werden kann und durch die entsprechende Bemessung der konischen Bohrung ohne Befestigungsmittel darauf sitzt. Um einen Flächenkontakt zwischen dem Kühlelement 2 und der Außenwandung des Kühlabschnittes 3 über die ganze Erstreckung zu gewährleisten und den Wärmeübergangswiderstand möglichst klein zu halten, ist auf der Außenseite des Kühlabschnittes 3 eine Wärmeleitpaste (nicht dargestellt) aufgetragen.

Beim Einsatz der erfindungsgemässen Trennvorrichtung, beispielsweise im Rahmen einer Gasanalyse-Anlage, steht der Gaseinlaßstutzen 7 über eine nicht gezeigte Leitung mit einem Gaskanal od.dgl. in Verbindung, dessen Gaszusammensetzung analysiert werden soll, während der Gasauslaßstutzen 5 an weitere Komponenten der Gasanalyse-Anlage angeschlossen ist. Der Kondensatablaufstutzen 4 ist mit einer nicht gezeigten Kondensatfalle verbunden, die so ausgebildet ist, daß die darin befindliche Kondensatansammlung keine freie Grenzfläche zu dem in dem Kühlrohr 1 strömenden Gas bildet (vgl. hierzu DE-GBM 87 07 036). Durch eine nicht gezeigte Saugpumpe wirkt an dem Gasauslaßstutzen 5 ein Unterdruck, der zu einer Einströmung des Gases durch die Einlaßöffnung 8 in tangentialer Richtung führt. Das einströmende Gas hat z.B. eine Temperatur von 60-70°C und bildet innerhalb des Kühlabschnittes 3 nach oben eine Schraubenströmung aus, bevor es durch den Gasauslaßstutzen 5 abgezogen wird. Hierdurch kommt es in innigen Kontakt mit der gekühlten kegeligen Mantelfläche des Kühlabschnittes 3, so daß es sich abkühlt und am Gasauslaß 5 mit einer Temperatur von z.B. 5°C austritt. Infolge der Abkühlung kondensiert der im Gas enthaltene Wasserdampf an der gekühlten Wandung in Form eines dünnen Kondensatfilmes, der nach unten fließt, in den Boden 6 gelangt und von dort über den Kondensatablaufstutzen abgeleitet wird.

Die Fig. 1, 2 zeigen ein praktisches Ausführungsbeispiel des Kühlrohres nach der Erfindung im Maßstab 1 : 1, dessen Volumen etwa 25cm$^3$ bei einer Innen-Oberfläche von etwa 40cm$^2$ beträgt. Diese Innen-Oberfläche ist die rechnerische Kühlfläche, die in der Praxis voll ausgenützt werden kann, womit sie weniger als ein Drittel der Innen-Oberfläche der eingangs geschilderten bekannten Trennvorrichtung bei gleicher Abscheideleistung ausmacht. Das Material des Kühlrohres ist zweckmässigerweise Glas.

Es versteht sich, daß im Rahmen des Erfindungsgedankens von den Details des vorstehenden Ausführungsbeispiels abgewichen werden kann. So kann je nach der Intensität der Kühlwirkung und des Wärmeüberganges der Konuswinkel α grösser oder kleiner als 5° eingestellt werden. Auch ist die senkrecht zur Achse des Kühlrohres 1 gerichtete Anordnung des Gaseinlaßstutzens 7 nicht kritisch, und auch eine zur Vertikalen geneigte Anordnung des Kühlrohres 1 ist im Sinne der Erfindung als aufrechte Anordnung zu verstehen, solange gewährleistet ist, daß unter dem Einfluß der Schwerkraft das sich an der Kühlrohrinnenwandung ausbildende Kondensat nach unten zum Kondensatablaufstutzen 4 gelangt. Weiterhin muß die Kühlung der Außenwand des Kühlabschnittes 3 nicht zwingend durch einen direkten Flächenkontakt mit einem Kühlelement erfolgen, wenn die gestellten Anforderungen an die Kühlwirkung geringer als beispielsweise im Fall der Gasaufbereitung für die Gasanalyse sind. So kann daran gedacht werden, bei geringeren Anforderungen beispielsweise eine Strömungskühlung durch einen Kühlluftstrom od.dgl. zu bewirken. Auch in diesem Fall reagiert die erfindungsgemässe Trennvorrichtung in ihrer Abscheidewirkung bzw. der Einsteuerung der am Gasauslaß 5 gemessenen Gastemperatur sehr rasch und ohne ins Gewicht fallende Regelschwankungen auf Änderungen in der Kühlintensität. Aus diesem Grund ist es möglich, schon innerhalb einer sehr kurzen Anlaufzeit und ohne die Gefahr eines Einfrierens der Trennvorrichtung am Gasauslaß die gewünschte Temperatur des Gases durch Veränderung der Kühlleistung einzustellen. Obwohl, wie oben erläutert, die senkrecht zur Achse des Kühlrohres 1 gerichtete Anordnung des Gaseinlaßstutzens 7 nicht kritisch ist, solange die geschilderte Schraubenströmung des Gasstromes erreicht wird, ergibt eine rechtwinkelige Anordnung den besten Effekt. Denn dadurch kommt das Gas nach seiner Einleitung in das Kühlrohr 1 in sofortigen Kontakt mit der Kühlrohrwandung an deren grösstem Durchmesser, so daß die größtmögliche Kondensatmenge sofort und dementsprechend am unteren Ende des Kühlabschnittes 3 ausfällt. Folglich ergibt sich in dem oberen Bereich aus der restlichen Kondensatmenge die erwähnte geringere Dicke des Kondensatfilmes mit den geschilderten Vorteilen und die am unteren Ende des Kühlabschnittes ausgefallene Kondensatmenge gelangt auf kürzestem Weg in den Kondensatablaufstutzen 4.

**Patentansprüche**

1. Vorrichtung zur Abscheidung dampfförmiger Bestandteile aus einem Gasstrom durch Kondensation bei der Gasaufbereitung, insbesondere für die Gasanalyse, mit einem annähernd aufrecht stehenden, von dem Gasstrom durchströmten Kühlrohr mit gekühlter Außenwandung, das einen Gaseinlaß und einen Gasauslaß sowie einen unten liegenden Kondensatablaufstutzen aufweist, dadurch gekennzeichnet, daß der Gaseinlaß (7, 8) im unteren Endabschnitt des Kühlrohres (3, 1) tangential bezüglich des Kühlrohrquerschnittes einmündet und daß das Kühlrohr (3, 1) sich von dem Gaseinlaß (7, 8) zu dem am oberen Ende des Kühlrohres angeordneten Gasauslaß (5) hin verjüngt.

2. Vorrichtung nach Anspruch 1, dadurch gekennzeichnet, daß das Kühlrohr (3, 1) sich nach oben konisch verjüngt.

3. Vorrichtung nach Anspruch 2, dadurch gekennzeichnet, daß der Konuswinkel etwa 5° beträgt.

4. Vorrichtung nach einem der Ansprüche 1 bis 3, dadurch gekennzeichnet, daß der Kondensatablaufstutzen (4) unmittelbar unter der Gaseinlaßmündung (8) angeordnet ist.

5. Vorrichtung nach einem der Ansprüche 1 bis 4, dadurch gekennzeichnet, daß der gekühlte Abschnitt (3) des Kühlrohres an seinem unteren Ende sich nach unten muldenförmig oder konisch (bei 8) verjüngt und der Kondensatablaufstutzen (4) koaxial zu dem Kühlrohr (1) daran anschließt.

6. Vorrichtung nach einem der Ansprüche 1 bis 5, dadurch gekennzeichnet, daß die Außenwandung des gekühlten Abschnittes (3) durch einen direkt aufsitzenden metallischen Kontaktblock (2) mit einem Peltier-Element gekühlt ist.

7. Vorrichtung nach einem der Ansprüche 1 bis 6, dadurch gekennzeichnet, daß der Gaseinlaß (7, 8) durch einen etwa rechtwinkelig zur Achse des Kühlrohres (1) angeordneten Gaseinlaßstutzen gebildet ist.

8. Vorrichtung nach Anspruch 7, dadurch gekennzeichnet, daß der Strömungsquerschnitt des Gaseinlaßstutzens (7) erheblich kleiner als der Kühlrohrquerschnitt im Bereich der Einlaßmündung (8) ist.

**Claims**

1. A device for separating vaporous components out of a flow of gas by condensation in the treatment of gas, in particular for gas analysis, comprising an approximately upright cooling tube through rich the flow of gas passes, with a cooled outside wall, the cooling tube having a gas inlet and a gas outlet and a condensate drain connection at the bottom, characterised in that the gas inlet (7, 8) opens in the lower end portion of the cooling tube (3, 1) tangentially with respect to the cross-section of the cooling tube and that the cooling tube (3, 1) decreases from the gas inlet (7, 8) to the gas outlet (5) rich is disposed at the upper end of the cooling tube.

2. A device according to claim 1 characterised in that the cooling tube (3, 1) decreases in a conical configuration upwardly.

3. A device according to claim 2 characterised in that the cone angle is about 5°.

4. A device according to one of claims 1 to 3 characterised in that the condensate drain connection (4) is disposed directly beneath the gas inlet mouth opening (8).

5. A device according to one of claims 1 to 4 characterised in that the cooled portion (3) of the cooling tube decreases at its lower end downwardly in a bowl-like or conical configuration (at 8) and the condensate drain connection (4) joins thereto coaxially with respect to the cooling tube (1).

6. A device according to one of claims 1 to 5 characterised in that the outside wall of the cooled portion (3) is cooled by a metal contact block (2), rich is carried directly on the cooled portion, with a Peltier element.

7. A device according to one of claims 1 to 6 characterised in that the gas inlet (7, 8) is formed by a gas inlet connection disposed approximately at a right angle to the axis of the cooling tube (1).

8. A device according to claim 7 characterised in that the flow cross-section of the gas inlet connection (7) is considerably smaller than the cross-section of the cooling tube in the region of the inlet mouth opening (8).


**Revendications**

1. Dispositif pour la séparation de composants à l'état de vapeur à partir d'un courant gazeux par condensation lors de la préparation de gaz, en particulier pour l'analyse de gaz, comprenant un tube de refroidissement qui est disposé approximativement verticalement et est traversé par le courant gazeux et qui présente une paroi externe refroidie, une entrée de gaz et une sortie de gaz ainsi qu'un tuyau d'évacuation de produit de condensation situé en bas, caractérisé en ce que l'entrée de gaz (7, 8) débouche tangentiellement par rapport à la section transversale du tube de refroidissement, dans le tronçon d'extrémité inférieure du tube de refroidissement (3, 1) et en ce que le tube de refroidissement (3, 1) se rétrécit depuis l'entrée de gaz (7, 8) vers la sortie de gaz (5) qui est disposée à l'extrémité supérieure du tube de refroidissement.

2. Dispositif suivant la revendication 1, caractérisé en ce que le tube de refroidissement (3, 1) se rétrécit de manière conique vers le haut.

3. Dispositif suivant la revendication 2, caractérisé en ce que l'angle du cône est d'environ 5°.

4. Dispositif suivant l'une des revendications 1 à 3, caractérisé en ce que le tuyau d'évacuation de produit de condensation (4) est agencé directement en dessous de l'embouchure (8) de l'entrée de gaz.

5. Dispositif suivant l'une des revendications 1 à 4, caractérisé en ce que le tronçon refroidi (3) du tube de refroidissement se rétrécit vers le bas sous la forme d'une cuvette ou d'un cône (en 8) à son extrémité inférieure et en ce que le tuyau d'évacuation de produit de condensation (4) s'y raccorde coaxialement au tube de refroidissement (1).

6. Dispositif suivant l'une des revendications 1 à 5, caractérisé en ce que la paroi externe du tronçon refroidi (3) est refroidie par un bloc de contact (2) métallique qui est en appui direct et qui présente un élément Peltier.

7. Dispositif suivant l'une des revendications 1 à 6, caractérisé en ce que l'entrée de gaz (7, 8) est formée par un tuyau d'entrée de gaz qui est agencé approximativement à angle droit par rapport à l'axe du tube de refroidissement (1).

8. Dispositif suivant la revendication 7, caractérisé en ce que la section transversale d'écoulement du tuyau d'entrée de gaz (7) est beaucoup plus petite que la section transversale du tube de refroidissement dans la zone de l'embouchure d'entrée (8).

Fig. 1

Fig. 2